**Europäisches Patentamt**

**European Patent Office**

**Office européen des brevets**

(11) Numéro de publication : **0 383 819 B1**

(19)

## (12) FASCICULE DE BREVET EUROPEEN

(45) Date de publication du fascicule du brevet :
**30.09.92 Bulletin 92/40**

(21) Numéro de dépôt : **88909776.2**

(22) Date de dépôt : **28.10.88**

(86) Numéro de dépôt international :
**PCT/FR88/00530**

(87) Numéro de publication internationale :
**WO 89/04156 18.05.89 Gazette 89/11**

(51) Int. Cl.$^5$ : **A61F 5/455**

(54) **RECEPTACLE COLLECTEUR PERMANENT D'URINE POUR FEMME.**

(30) Priorité : **30.10.87 FR 8715093**

(43) Date de publication de la demande :
**29.08.90 Bulletin 90/35**

(45) Mention de la délivrance du brevet :
**30.09.92 Bulletin 92/40**

(84) Etats contractants désignés :
**AT BE CH DE FR GB IT LI LU NL SE**

(56) Documents cités :
**EP-A- 0 185 517**
**US-A- 4 583 983**

(73) Titulaire : **KILRUSH LIMITED**
**49 Conduit Street**
**London W1R 9FB (GB)**

(72) Inventeur : **NIGAY, Pierre**
**10, rue de Paris**
**F-60200 Compiègne (FR)**

(74) Mandataire : **Bertrand, Didier et al**
**c/o S.A. FEDIT-LORIOT & AUTRES CONSEILS**
**EN PROPRIETE INDUSTRIELLE 38, Avenue**
**Hoche**
**F-75008 Paris (FR)**

## Description

L'invention concerne un réceptacle collecteur permanent d'urine pour femme.

Le document EP-A-148 047 et le document EP-A-287 441 font connaître des équipements destinés aux personnes incontinentes et permettant la détection et le pompage de l'urine captée au niveau des voies urinaires pour la stoker dans une poche souple, généralement retenue à la ceinture.

Ces équipements donnent satisfaction. Cependant, dans certains cas, le captage de l'urine est défectueux, surtout dans certaines positions du sujet et notamment la position couchée. D'autre part, en cas de double incontinence urinaire et fécale, il arrive que les pertes fécales viennent dérégler le fonctionnement de ces équipements.

L'incontinence féminine est plus répandue que l'incontinence masculine, et des réceptacles collecteurs pour femmes améliorés ont déjà été proposés.

On connaît ainsi des dispositifs de drains urinaires portés en permanence par le sujet. Le document US-A-4,583,983 montre un tel drain, comportant un plateau et une cheminée oblongue biseautée destinée à coopérer avec l'urètre, au niveau du méat urinaire, pour collecter l'urine, laquelle est ensuite évacuée directement par gravité dans une poche souple. Si ce drain peut donner satisfaction dans les cas les plus simples, c'est-à-dire lorsque les organes féminins sont régulièrement conformés et quand le sujet est en position verticale, il est clair qu'il n'est pas satisfaisant quand le sujet est en position couchée et/ou lorsque l'anatomie de la région urinaire est perturbée par exemple à la suite d'une descente d'organe.

On connaît par ailleurs des récipients collecteurs provisoires, sortes d'urinaux. Par exemple, le document EP-A-185 517 montre un tel urinal, destiné à être utilisé exclusivement provisoirement, par un sujet couché. Ceci est tout à fait différent des réceptacles collecteurs permanents visés par l'invention présente.

Le but de l'invention est de préparer un réceptacle permanent, exempt des inconvénients précités, particulièrement adapté à être utilisé avec les équipements de détection et de pompage cités en tête de ce mémoire.

L'invention atteint son but en proposant un réceptacle à plateau et cheminée oblongue biseautée, dans lequel le plateau comporte une lèvre relevée souple conformée pour entourer les organes féminins, dans la zone intérieure de laquelle se trouve ladite cheminée, entourée d'une rigole et, sur un côté seulement, d'une cuvette ; la cheminée et la rigole communiquent avec une enceinte collectrice attenante au plateau, le réceptacle étant formé d'un matériau semi-souple.

La lèvre relevée assure, grâce à une certaine souplesse, une parfaite étanchéité entre la zone de captage et l'extérieur, aussi bien dans un sens que dans l'autre. D'une part, quelle que soit la position du sujet féminin, l'urine émise est recueillie, soit par la cheminée dans le cas le plus normal, soit par la cuvette et la rigole dans les autres cas, sans fuite vers l'extérieur. D'autre part, la partie arrière de la lèvre offre une barrière aux éventuelles pertes fécales qui pourraient venir pertuber le fonctionnement du dispositif.

L'enceinte collectrice comporte des moyens d'évacuation, qui peuvent être un orifice d'écoulement gravitaire relié par exemple à une poche réceptrice située plus bas ; plus avantageusement, ces moyens sont associés à un système de détection et de pompage, par exemple à celui des brevets cités en tête de ce mémoire.

L'évacuation est alors avantageusement prévue à la partie supérieure de l'enceinte.

Le réceptacle est de préférence obtenu par moulage "flow-casting" en un matériau semi-souple, tel qu'un latex naturel dont la souplesse permet de ne pas agresser les organes féminins et d'en épouser plus facilement le contour.

D'autres caractéristiques et avantages de l'invention ressortiront de la description suivante, d'un mode de réalisation préféré. On se réfère aux dessins annexés sur lesquels :

– la figure 1 est une vue en perspective de 3/4 du réceptacle de l'invention ;
– la figure 2 est une vue de dessus du même réceptacle,
– la figure 3 en est une vue latérale, avec arrachement partiel.

Le réceptacle 1 est une pièce semi-rigide semi-souple avantageusement en latex naturel et obtenue par moulage.

Elle comporte un plateau 2, en triangle isocèle inversé, dont la base supérieure comporte deux ouvertures 3 destinées au passage de sangles avant de suspension.

Au-dessus du plateau 2, s'élève une lèvre d'étanchéité 4, relativement souple, dont le contour fermé est destiné à entourer les organes féminins. Le lèvre 4 s'évase légèrement vers l'extérieur.

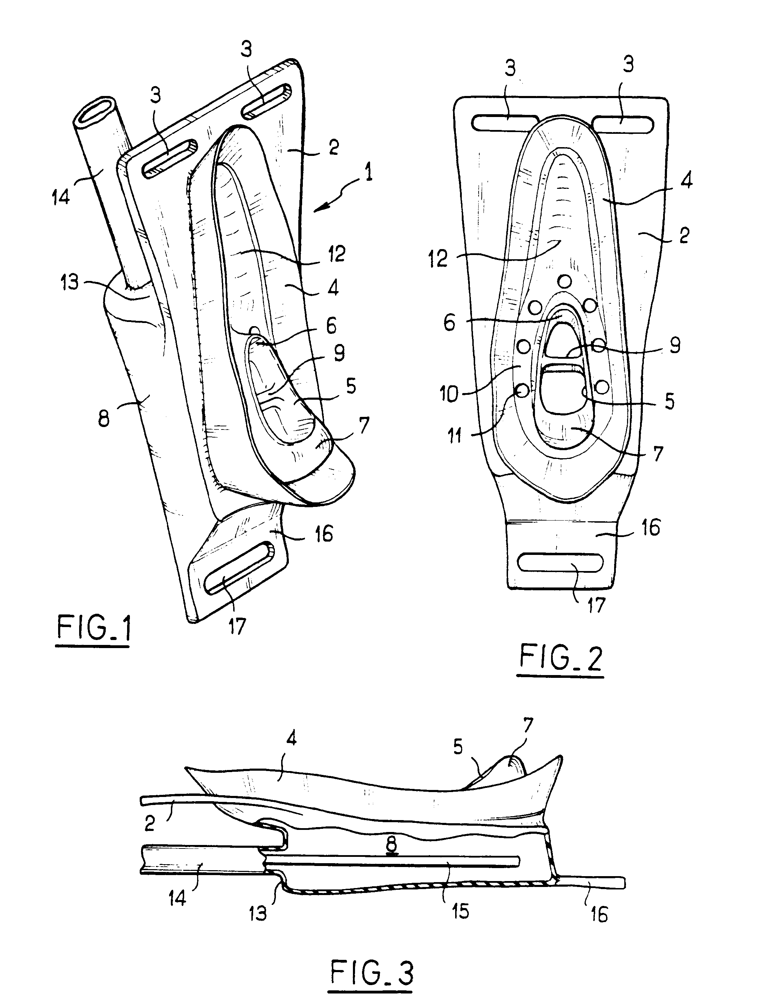

A l'intérieur de cette lèvre 4, en position excentrée inférieure, est prévue une cheminée creuse de captage 5, de forme oblongue (en vue de dessus ; cf. figures 1 et 2) et biseautée (en vue latérale ; cf. figures 1 et 3), sa partie avant 6 étant moins élevée qui sa partie arrière 7. La cheminée s'enfonce à travers le plateau 2 et communique avec une enceinte collectrice de réception 8, allongée, formée sous le plateau 2 et attenante à lui.

La cheminée 5 possède entre ses deux bords sensiblement parallèles une cloison intermédiaire 9 qui évite le resserrement desdits bords.

Au pied de la cheminée 5, tout autour d'elle, une rigole 10 peut recueillir et acheminer le liquide vers l'enceinte 8 au travers d'orifices 11 prévus dans le fond de la rigole. Les orifices 11 servent d'évents lors du remplissage de l'enceinte 8 par la cheminée 5.

Vers la partie avant supérieure de la zone intérieure à la lèvre 4, une cuvette allongée 12 peut recueillir le liquide et le diriger vers la rigole 10.

En dessous du plateau 2, l'enceinte 8 est pourvue, sur une paroi frontale avant supérieure 13, d'un tube d'évacuation 14 relié à l'ensemble de pompage décrit dans les brevets précités. On a représenté sur la figure 3, l'extrémité du tuyau d'aspiration 15.

La forme de l'enceinte 8, et la position du tuyau d'aspiration 15 et des électrodes de détection sont avantageusement prévues pour conserver dans la poche une petite quantité permanente de liquide qui évite des phénomènes de bruit liés à la présence d'air dans les circuits de pompage.

Au bas de l'enceinte 8 est prévue une patte 16 pourvue d'une fente 17 par le passage d'une sangle inférieure de fixation. Le cas échéant, cette patte de support 16 peut être reliée à un dispositif de rétention des selles qui fait suite au dispositif collecteur d'urine.

## Revendications

1. Réceptacle collecteur permanent pour recueillir l'urine d'une femme, du type comportant un plateau et une cheminée oblongue biseautée destinée à coopérer avec l'urètre, caractérisé en ce que le plateau comporte une lèvre relevée souple (4) conformée pour entourer les organes féminins, dans la zone intérieure de laquelle se trouve ladite cheminée (5), entourée d'une rigole (10), et, d'un côté seulement, d'une cuvette (12), la cheminée (5) et la rigole (10) communiquant avec une enceinte collectrice (8) attenante au plateau, le réceptacle étant formé d'un matériau semi-souple.

2. Réceptacle selon la revendication 1, caractérisé en ce que l'enceinte collectrice (8) comporte des moyens d'évacuation (14, 15).

3. Réceptacle selon la revendication 2, caractérisé en ce.que les moyens d'évacuation sont associés à un système de pompage.

## Patentansprüche

1. Permanenter Sammelbehälter zum Auffangen des Urins einer Frau, der Art, die über eine Platte und einen länglichen, abgeschrägten Kaminabzug verfügt, der zur Zusammenarbeit mit der Harnröhre bestimmt ist, dadurch gekennzeichnet, daß die Platte über eine hochgezogene weiche Lippe (4) verfügt, die so gestaltet ist, daß sie die weiblichen Organe umgibt, in deren inneren Zone sich der Kaminabzug (5) befindet, der von einem Abzugkanal (10) und auf lediglich einer Seite von einer Küvette (12) umgeben ist, wobei der Kaminabzug (5) und der Abzugkanal (10) mit einem an die Platte anstoßenden Sammelraum (8) in Verbindung stehen, wobei der Behälter aus einem halbweichen Stoff gebildet ist.

2. Behälter nach Anspruch 1, dadurch gekennzeichnet, daß der Sammelraum (8) über Evakuierungsmittel (14, 15) verfügt.

3. Behälter nach Anspruch 2, dadurch gekennzeichnet, daß die Evakuierungsmittel an ein Pumpensystem angeschlossen sind.

## Claims

1. A permanent urine-collecting receptacle for women, of the type having a plate and a beveled oblong duct for cooperating with the urethra, characterized in that the plate has a flexible raised lip (4) shaped so as to surround the female organs, the said duct (5) being located in the interior zone of the said lip and being surrounded by a channel (10) and, on one side only, by a dish (12), and the duct (5) and the channel (10) communicating with a collecting chamber (8) adjoining the plate, the receptacle being made from a semi-flexible material.

2. The receptacle according to claim 1, characterized in that collecting chamber (8) has discharge means (14, 15).

3. The receptacle according to claim 2, characterized in that the discharge means are associated with a pumping system.

FIG. 1

FIG. 2

FIG. 3